# EUROPEAN PATENT SPECIFICATION

(11) **EP 2 679 158 B1**
(45) Date of publication and mention of the grant of the patent: **10.06.2020**
(21) Application number: 13166729.7
(22) Date of filing: 07.05.2013
(51) Int. Cl.: A61B 6/00

(54) **Method and apparatus for displaying ultrasonic image and information related to the ultrasonic image**
Verfahren und Vorrichtung zum Anzeigen von Ultraschallbildern und Informationen bezüglich des Ultraschallbildes
Procédé et appareil pour afficher une image ultrasonore et des informations relatives à l'image ultrasonore

(30) Priority: 01.06.2012 KR 20120059430
(43) Date of publication of application: 01.01.2014
(73) Proprietor: Samsung Medison Co., Ltd., Gangwon-do 250-870 (KR)
(72) Inventor: Lee, Jin-yong, Hongcheon-gun, Gangwon-do (KR); Lee, Bong-heon, Hongcheon-gun, Gangwon-do (KR); Park, Sung-wook, Hongcheon-gun, Gangwon-do (KR)
(74) Representative: Lorenz, Markus

(56) References cited:
- US-A1- 2008 051 652
- US-A1- 2008 077 013
- US-A1- 2010 036 248
- US-A1- 2011 087 094

## Description

### BACKGROUND OF THE INVENTION

### 1. Field of the Invention

The present invention relates to a method and apparatus for displaying an ultrasonic image and information associated with the ultrasonic image, and more particularly, to a method and apparatus for displaying an ultrasonic image and information associated with the ultrasonic image together based on cross-section information of the ultrasonic image.

### 2. Description of the Related Art

Ultrasonic diagnosis apparatuses transmit an ultrasonic signal to a predetermined part of the inside of a target object via the body surface of the target object and obtain an image associated with the fault or blood flow of soft tissue of the target object by using information of an ultrasonic signal reflected by tissue of the inside of the target object.

Such ultrasonic diagnosis apparatuses are capable of displaying an image of a target object in real time. In addition, such ultrasonic diagnosis apparatuses are very safe because there is no radiation exposure by X rays or the like, and thus are widely used together with other image diagnosis apparatuses, such as an X-ray diagnosis apparatus, a computed tomography (CT) scanner, a magnetic resonance imaging (MRI) apparatus, and a nuclear medicine diagnosis apparatus.

The shape or the like of the internal tissue or the like of a target object may very slightly vary depending on the person, but the locations or internal structures of the internal tissues of people are mostly similar to one another. Accordingly, a standard cross-section image may be acquired from similar scanned cross-sections of a target object.

When ultrasonic diagnosis apparatuses use information about such a standard cross-section image, a user may be easily aware of what part of a target object has been scanned. Moreover, a current state or the like of a scanned tissue may also be clearly understood via comparison with information about the standard cross-section image.

US 2010/036248 A1 describes a medical image diagnostic apparatus which can mitigate an operational burden at the time when measurement processing is performed by use of a medical image. The ultrasonic diagnostic apparatus acquires input image information, which is image data of a subject, and holds it in a storage section. An image selection section performs image recognition calculation for comparison between the input image information and past image information pieces of an image measurement database.

### SUMMARY OF THE INVENTION

The present invention provides a method and apparatus for displaying an ultrasonic image and information associated with the ultrasonic image, according to claims 5 and 1, respectively.

According to an aspect of the present invention, there is provided an apparatus for displaying an ultrasonic image and information associated with the image, the apparatus including a storage unit which stores a plurality of cross-section images of a plurality of scanned cross-sections of a target object and a plurality of pieces of image-related information respectively associated with the plurality of cross-section images, an image acquisition unit which acquires an ultrasonic image of the target object, a selection unit which selects at least one of the plurality of cross-section images based on a similarity between the ultrasonic image and each of the plurality of cross-section images, an extraction unit which extracts image-related information associated with the selected cross-section image from the storage unit, and a display unit which displays the extracted image-related information and the acquired ultrasonic image.

The plurality of cross-section images may include respective standard cross-section images for a plurality of standard cross-sections.

The image-related information pieces may include at least one of information about a scan method of the target object, information about a biopsy of the target object, information about a measurement of the target object, and information about display of the target object.

The selection unit includes a comparison unit which determines a similarity between the acquired ultrasonic image and each of the plurality of cross-section images by comparing the acquired ultrasonic image with each of the plurality of cross-section images.

The selected cross-section image is a cross-section image having a highest similarity to the acquired ultrasonic image from among the plurality of cross-section images.

The comparison unit may include an alarm unit which produces an alarm message when the determined similarity is less than or equal to a predetermined standard level.

The alarm message may be a message that advises the apparatus to re-acquire the ultrasonic image of the target object, or a message that advises the apparatus to adjust the resolution of the ultrasonic image.

The extraction unit includes a determination unit which determines the priorities of the image-related information pieces based on relations of the image-related information pieces with the selected cross-section image.

The extraction unit extracts the image-related information pieces according to the determined priorities.

The display unit may display the extracted image-related information and the acquired ultrasonic image together.

According to another aspect of the present invention, there is provided a method of displaying an ultrasonic image and information associated with the image, the method comprising storing a plurality of cross-section images of a plurality of scanned cross-sections of a target object and a plurality of pieces of image-related information respectively associated with the plurality of cross-section images, acquiring an ultrasonic image of the target object, selecting at least one of the plurality of cross-section images based on a similarity between the ultrasonic image and each of the plurality of cross-section images, extracting image-related information associated with the selected cross-section image, and displaying the extracted image-related information and the acquired ultrasonic image together.

The plurality of cross-section images may include respective standard cross-section images for a plurality of standard cross-sections.

The image-related information pieces includes at least one of information about a scan method of the target object, information about a biopsy of the target object, information about a measurement of the target object, and information about display of the target object.

The selecting includes determining a similarity between the acquired ultrasonic image and each of the plurality of cross-section images by comparing the acquired ultrasonic image with each of the plurality of cross-section images.

The selected cross-section image is a cross-section image having a highest similarity to the acquired ultrasonic image from among the plurality of cross-section images.

The determining may include producing an alarm message when the determined similarity is less than or equal to a predetermined standard level.

The alarm message may be a message that advises the ultrasonic image of the target object to be re-acquired, or a message that advises the resolution of the ultrasonic image to be adjusted.

The extracting includes determining the priorities of the image-related information pieces based on relations of the image-related information pieces with the selected cross-section image.

The image-related information pieces is extracted according to the determined priorities.

The displaying may include displaying the extracted image-related information and the acquired ultrasonic image together.

According to another aspect of the present invention, there is provided a computer-readable recording medium having recorded thereon a program for executing the method.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of an apparatus for displaying an ultrasonic image and information associated with the ultrasonic image, according to an embodiment of the present invention;
FIG. 2A illustrates a plurality of cross-section images of a plurality of scanned cross-sections of a target object, according to an embodiment of the present invention;
FIG. 2B illustrates an ultrasonic image that is acquired with respect to a target object, according to an embodiment of the present invention;
FIG. 3 is a block diagram of a selection unit included in the apparatus illustrated in FIG. 1, according to an embodiment of the present invention;
FIG. 4 is a block diagram of an extraction unit included in the apparatus illustrated in FIG. 1, according to an embodiment of the present invention;
FIG. 5 is a flowchart of a method of displaying an ultrasonic image and information associated with the ultrasonic image, according to an embodiment of the present invention;
FIG. 6 illustrates a display of an ultrasonic image and information associated with the ultrasonic image, according to an embodiment of the present invention;
FIG. 7 illustrates a display of an alarm message, according to an embodiment of the present invention;
FIG. 8A illustrates an example of selection of information associated with an ultrasonic image;
FIG. 8B illustrates a display of image-related information according to the priority of image-related information, according to an embodiment of the present invention;
FIG. 9A illustrates another example of selection of information associated with an ultrasonic image; and
FIG. 9B illustrates selection and display of information associated with an ultrasonic image, according to an embodiment of the present invention.

### DETAILED DESCRIPTION OF THE INVENTION

Expressions such as "at least one of" when preceding a list of elements, modify the entire list of elements and do not modify the individual elements of the list.

Terminology used herein will now be briefly described, and the present invention will be described in detail.

Although general terms being widely used at present were selected as terminology used in the present invention while considering the functions of the present invention, they may vary according to intentions of one of ordinary skill in the art, judicial precedents, the advent of new technologies, and the like. Terms arbitrarily selected by the applicant of the present invention may also be used in a specific case. In this case, their meanings need to be given in the detailed description of the present invention. Hence, the terms must be defined based on the meanings of the terms and the contents of the entire specification, not by simply stating the terms themselves.

It will be understood that the terms "comprises" and/or "comprising" or "includes" and/or "including" when used in this specification, specify the presence of stated elements, but do not preclude the presence or addition of one or more other elements. Terms such as "... unit" and "module" stated in the specification denote units that process at least one function or operation, and they may be implemented by using hardware, software, or a combination of hardware and software.

In the entire specification, an "ultrasonic image" denotes an image of a target object that is acquired using ultrasounds. The target object may denote a part of a human body. For example, the target object may be an organ, such as a liver, a heart, a womb, a brain, a breast, or an abdomen, or a fetus.

In the entire specification, a "user" may be a medical expert, such as a doctor, a nurse, a medical technologist, or a medical image expert, but the present invention is not limited thereto.

The present invention will now be described more fully with reference to the accompanying drawings, in which exemplary embodiments of the invention are shown. The invention may, however, be embodied in many different forms and should not be construed as being limited to the embodiments set forth herein. In the drawings, parts irrelevant to the description are omitted for simplicity of explanation, and like numbers refer to like elements throughout.

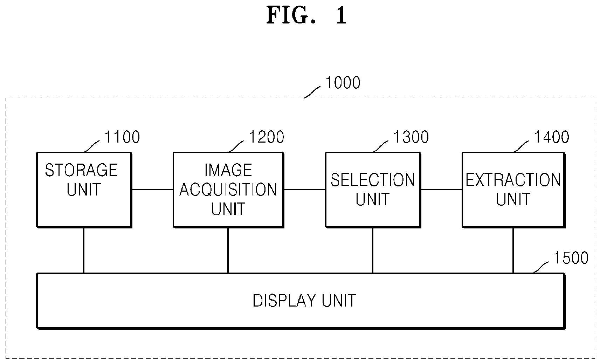

FIG. 1 is a block diagram of an apparatus 1000 for displaying an ultrasonic image and information associated with the ultrasonic image, according to an embodiment of the present invention.

Referring to FIG. 1, the apparatus 1000 may include a storage unit 1100, in which a plurality of cross-section images of a plurality of scanned cross-sections of a target object and pieces of image-related information respectively associated with the cross-section images are stored, an image acquisition unit 1200, which acquires an ultrasonic image of the target object, and a selection unit 1300, which selects at least one of the cross-section images based on a similarity between the acquired ultrasonic image and each of the cross-section images.

The apparatus 1000 may further include an extraction unit 1400, which extracts image-related information associated with the selected cross-section image from the storage unit 1100, and a display unit 1500, which displays the extracted image-related information and the acquired ultrasonic image.

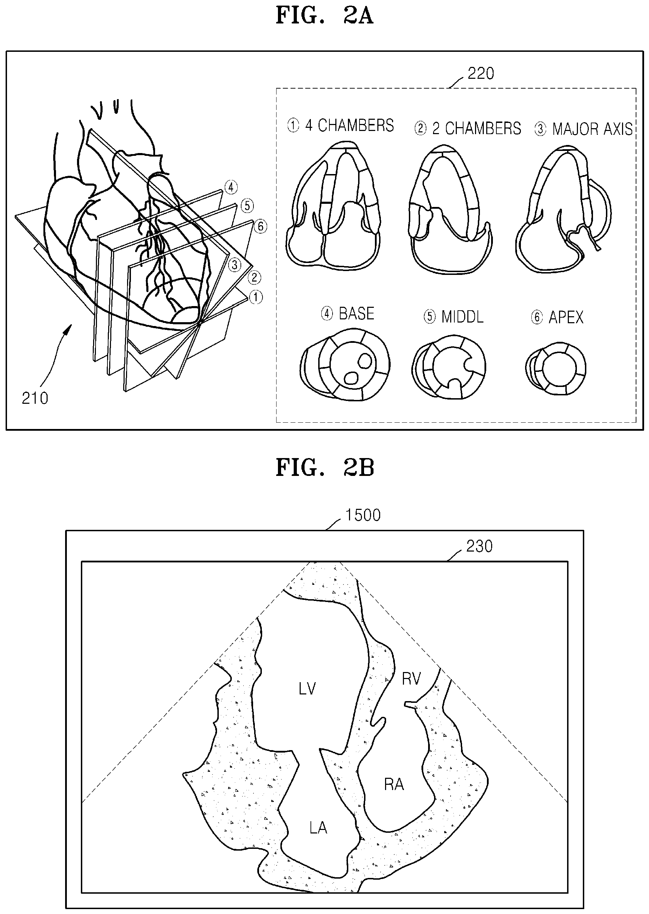

FIG. 2A illustrates a plurality of cross-section images 220 of a plurality of scanned cross-sections of a target object 210, according to an embodiment of the present invention. FIG. 2B illustrates an ultrasonic image 230 that is acquired with respect to the target object 210, according to an embodiment of the present invention.

The cross-section images 220 may include standard cross-section images for a plurality of standard cross-sections. The standard cross-sections may include cross-sections that include identifiable characteristics for different types and different parts of the target object 210. The identifiable characteristics may include feature vectors or the like of the standard cross-sections.

For example, when the target object 210 is a heart as illustrated in FIG. 2A, the standard cross-sections may include a parasternal section, an apical section, a subcostal section, a suprasternal section, and the like.

In other words, when the target object 210 is a heart of a human being, the shapes or the like of hearts may be partly different depending on the person, but the overall structures and shapes of the hearts of people may be similar to each other.

For example, the heart of a person generally includes two atria and two ventricles, and a standard cross-section image ① including such two atria and two ventricles, namely, four chambers, may be acquired as illustrated in FIG. 2A. A plurality of pixel values included in the standard cross-section image may be expressed as an NxM matrix, and this matrix may be indicated as a feature vector of the standard cross-section image. N and M may be equal to each other or different from each other.

Image-related information according to an embodiment of the present invention may include at least one of information about a scan method of a target object, information about a biopsy of the target object, information about a measurement of the target object, and target object display information.

The scan method, the biopsy, and the measuring method of the target object may vary depending on what the target object 210 is. For example, a scan method used when the target object 210 is a fast-moving target object such as a heart may be different from that used when the target object 210 is a relatively static target object such as a liver.

Accordingly, provision of a scan method or the like suitable for a cross-section image of a scanned target object may enable efficient scanning, an efficient biopsy, or the like to be performed consistently regardless of how much a user is skilled at performing a scanning technique.

For example, the scan method information may include information about the order of scanning a target object, information about the angle at which the target object is scanned, and the like.

For example, the biopsy information may include information about the order in which a target object is inspected by using a biopsy needle or the like, information about attention points during the inspection, information about a method for the inspection, and the like.

The measurement according to an embodiment of the present invention may include measurement of the length, size, and area of a target object, measurement of the movement period of time of the target object, measurement of the inclination of the target object, and the like. The measurement may also include measurement of the speed at which the target object moves in a color mode of a scanner (not shown) that uses ultrasound. Accordingly, the measurement information may include information about measurement items for the above-described measurements and information about measuring standard scales for the above-described measurements.

The target object display information may include, for example, a text that may be input to an image obtained by scanning a target object, and a body marker that marks the image obtained by scanning a target object.

At least one of a plurality of cross-section images and a plurality of pieces of image-related information respectively associated with the cross-section images according to an embodiment of the present invention may be previously stored in the apparatus 1000.

Alternatively, at least one of the cross-section images and the image-related information pieces may be stored in real time in the apparatus 1000. For example, at least one of the cross-section images and the image-related information pieces may be edited, for example, added or changed, according to a user input.

For example, new information may be added to pre-stored image-related information according to an external user input to the apparatus 1000, or new image-related information may be stored in the storage unit 1100 of the apparatus 1000. Since this function for editing the image-related information may facilitate renewal or the like of the image-related information, operational convenience of a user who uses an ultrasonic apparatus may increase.

As illustrated in FIG. 2B, the ultrasonic image 230, which is obtained by scanning the target object 210, may be displayed on the display unit 1500.

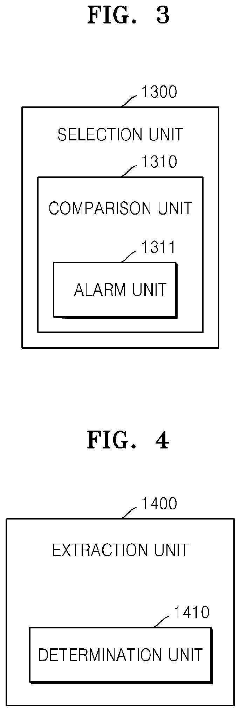

FIG. 3 is a block diagram of the selection unit 1300 according to an embodiment of the present invention.

The selection unit 1300 may include a comparison unit 1310, which compares the acquired ultrasonic image 230 with each of the cross-section images 220 to determine a similarity therebetween.

The cross-section image selected by the selection unit 1300 may be a cross-section image having a highest similarity from among the cross-section images 220.

For example, the ultrasonic image 230 including all of the four chambers as illustrated in FIG. 2B may be acquired by the image acquisition unit 1200. Accordingly, when the pixel values of a plurality of pixels included in the ultrasonic image 230 are compared with those of a plurality of pixels included in each of the cross-section images 220, the standard cross-section image ① including four chambers may have the greatest similarity to the ultrasonic image 230.

In other words, the selection unit 1300 may select the standard cross-section image ①, which is determined to have a highest similarity to the ultrasonic image 230 from among the cross-section images 220 based on a result of the comparison by the comparison unit 1310.

Referring back to FIG. 3, the comparison unit 1310 may include an alarm unit 1311, which produces an alarm message when the determined similarity is less than or equal to a predetermined standard level. The alarm message may be a message that advises the apparatus 1000 to re-acquire the ultrasonic image 230 of the target object.

For example, the predetermined standard level may include 50%. When a standard cross-section image corresponding to the acquired ultrasonic image 230 cannot be determined because the similarities between the acquired ultrasonic image 230 and each of the cross-section images 220 are all low according to a result of the comparison by the comparison unit 1310, the apparatus 1000 may produce the alarm message and display the alarm message on the display unit 1500.

For example, the alarm message may include a message that advises the apparatus 1000 to re-acquire the ultrasonic image 230, and a message that advises the apparatus 1000 to adjust the resolution of the ultrasonic image 230.

FIG. 4 is a block diagram of the extraction unit 1400 according to an embodiment of the present invention.

Referring to FIG. 4, the extraction unit 1400 may include a determination unit 1410, which determines the priorities of the image-related information pieces based on relations of the image-related information pieces with the selected cross-section image. The extraction unit 1400 may extract image-related information from the storage unit 1100 according to the determined priorities.

As exemplified above, when the standard cross-section image ① determined to have a highest similarity to the acquired ultrasonic image 230 is selected from the cross-section images 220, the priorities of the image-related information pieces may be determined according to relations with the standard cross-section image ①. In other words, image-related information having a high relation with a standard cross-section image may be determined to have a higher priority than the other image-related information pieces.

According to an embodiment, these relations may be preset and pre-stored in the storage unit 1100. For example, standard cross-section images and image-related information may be previously matched with each other by using an identifier that represents the degree of relation. This identifier may include an index, a tag, and the like that use numbers, characters, and the like.

For example, this relation may be stored in the form of a list or the like in which standard cross-section images are mapped with image-related information, in the storage unit 1100. For example, image-related information having a highest relation with the standard cross-section image ① from among the cross-section images 220 may represent its relation by using an identifier such as a numeral 1 or an alphabet letter A.

Such standard cross-section images and such image-related information may be mapped in a one-to-one correspondence, in a one-to-many correspondence, or in a many-to-many correspondence.

For example, if the number of image-related information pieces associated with a standard cross-section image is only one, the standard cross-section image may be mapped with the image-related information in a one-to-one correspondence. A plurality of image-related information pieces may be mapped with one standard cross-section image in a one-to-many correspondence while having the same relation with the standard cross-section image. A plurality of image-related information pieces may be mapped with one standard cross-section image in a one-to-many correspondence while having different relations with the standard cross-section image.

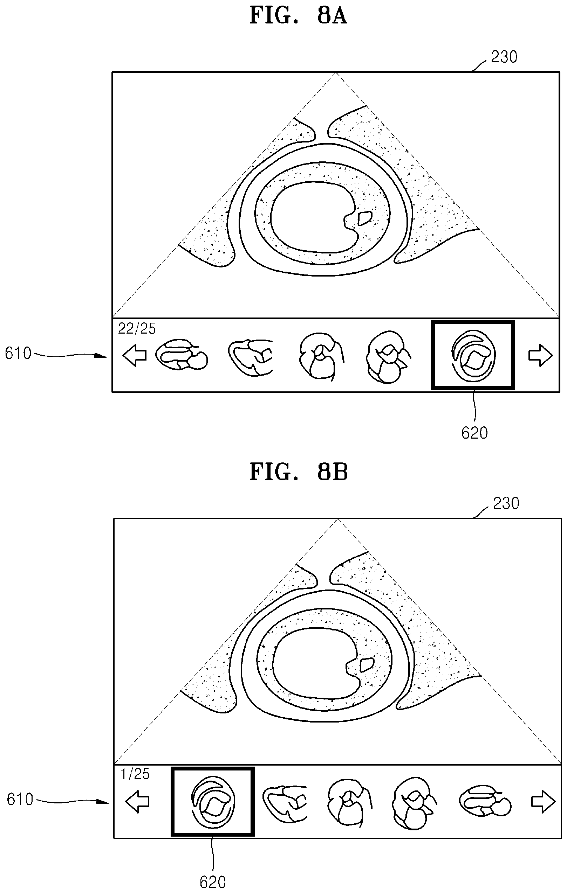

This will be described in detail later with reference to FIGS. 8A-9B.

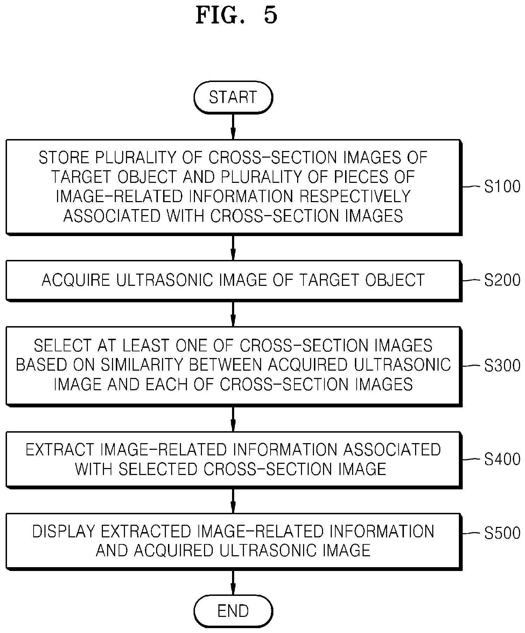

FIG. 5 is a flowchart of a method of displaying the ultrasonic image 230 and information associated with the ultrasonic image 230, according to an embodiment of the present invention.

Referring to FIG. 5, the method may include operation S100 of storing a plurality of cross-section images of a plurality of scanned cross-sections of a target object and a plurality of pieces of image-related information respectively associated with the cross-section images, operation S200 of acquiring the ultrasonic image 230 of the target object, and operation S300 of selecting at least one of the cross-section images based on a similarity between the acquired ultrasonic image 230 and each of the cross-section images.

The method may further include operation S400 of extracting image-related information associated with the selected cross-section image from the storage unit 1100, and operation S500 of displaying the extracted image-related information and the acquired ultrasonic image 230.

The cross-section images 220 may include respective standard cross-section images for a plurality of standard cross-sections.

The image-related information may include at least one of information about a scan method of a target object, information about a biopsy of the target object, information about a measurement of the target object, and target object display information.

At least one of the cross-section images 220 and the image-related information pieces respectively associated with the cross-section images 220 may be previously stored in the storage unit 1100. Alternatively, at least one of the cross-section images and the image-related information pieces may be stored in real time in the storage unit 1100.

Operation S300 may include determining a similarity between the acquired ultrasonic image 230 and each of the cross-section images 220 by comparing the acquired ultrasonic image 230 with each of the cross-section images 220. The cross-section image selected in operation S300 may be a cross-section image having a highest similarity to the acquired ultrasonic image 230 from among the cross-section images 220.

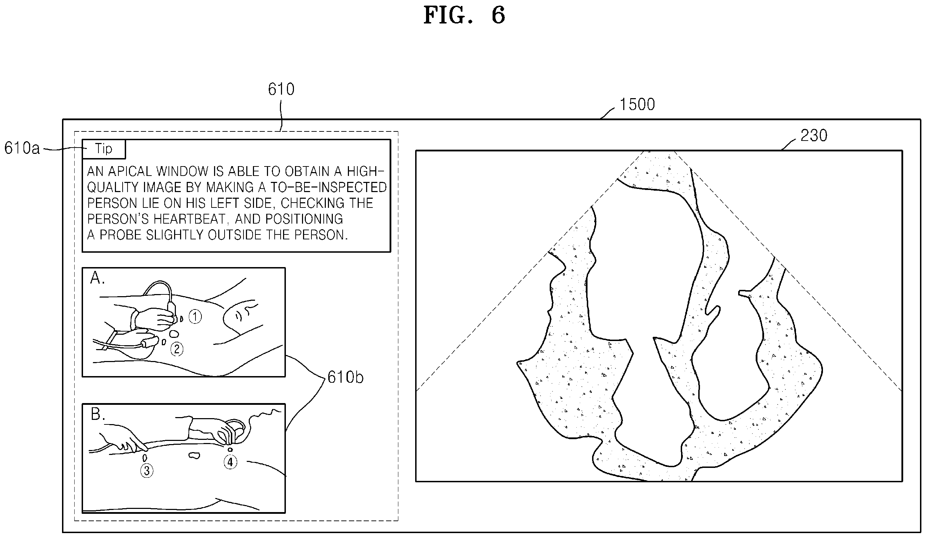

FIG. 6 illustrates a display of the ultrasonic image 230 and information associated with the ultrasonic image 230, according to an embodiment of the present invention.

Referring to FIGS. 2A and 6, the cross-section images 220 of the target object 210 and image-related information 610 associated with each of the cross-section images 220 may be stored. The ultrasonic image 230 may be acquired. A cross-section image having a highest similarity to the ultrasonic image 230 may be selected from the cross-section images 220. For example, when the ultrasonic image 230 is an image including 4 chambers as exemplified above, the standard cross-section image ① including 4 chambers may be selected from the cross-section images 220.

Image-related information 610 associated with the selected standard cross-section image ① may be extracted from the storage unit 1100 and may be displayed.

As described above, the extracted image-related information 610 may include information about a scan method of a target object, information about a biopsy sequence and a biopsy method for a biopsy of the target object, and so on, in order to obtain a high-quality image. The extracted image-related information 610 may further include information about a measurement of the target object and target object display information.

Referring to FIG. 6, the extracted image-related information 610 may be displayed using a text or the like as indicated by reference numeral 610a or may be displayed using a picture or a moving picture as indicated by reference numeral 610b. Alternatively, the extracted image-related information 610 may be displayed using at least one of a text and a moving picture.

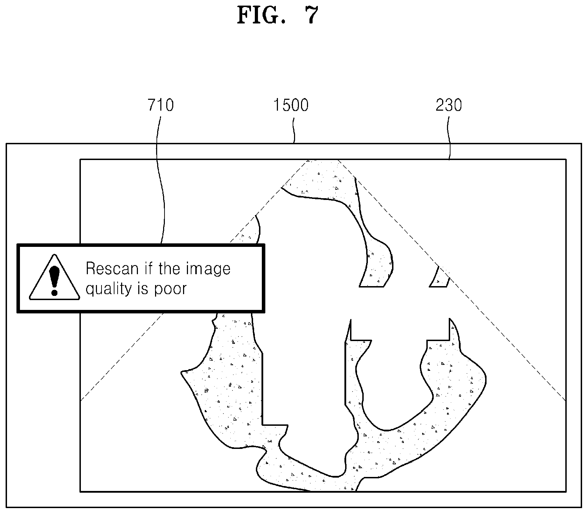

FIG. 7 illustrates a display of an alarm message 710, according to an embodiment of the present invention.

The aforementioned determining of the similarity may include producing the alarm message 710 when the determined similarity is less than or equal to a predetermined standard level. For example, the alarm message 710 may include a message that advises the apparatus 1000 to re-acquire the ultrasonic image 230, and a message that advises the apparatus 1000 to adjust the resolution of the ultrasonic image 230.

For example, the predetermined standard level may include about 50%. In other words, when a similarity between the standard cross-section image 220 and the ultrasonic image 230 is less than or equal to 50%, the alarm message 710 may be produced and displayed on the display unit 1500.

For example, when the acquired ultrasonic image 230 is compared with each of the cross-section images 220 and accordingly, it is difficult to interpret the acquired ultrasonic image 230, namely, when a standard cross-section image corresponding to the acquired ultrasonic image 230 cannot be determined, the alarm message 710 may be produced and displayed on the display unit 1500 of the apparatus 1000.

FIG. 8A illustrates an example of selection of information associated with an acquired ultrasonic image 230.

FIG. 8B illustrates a display of pieces of image-related information 610 according to the priorities of the image-related information pieces 610, according to an embodiment of the present invention.

Referring to FIG. 8A, the acquired ultrasonic image 230 and the image-related information pieces 610 are displayed. For example, a body marker included in target object display information may be displayed as each of the image-related information pieces 610.

Since the ultrasonic image 230 and the image-related information pieces 610 are displayed together, a user may easily select image-related information, for example, a body marker, suitable for the ultrasonic image 230.

Operation S400 of FIG. 5 may include determining the priorities of image-related information pieces based on the relations of the image-related information pieces with the selected cross-section image. The image-related information pieces may be extracted according to the determined priorities.

For example, the priorities of image-related information pieces suitable for the ultrasonic image 230 may be determined based on relations with the ultrasonic image 230. An image-related information piece 610 having the highest relation with the ultrasonic image 230 may be extracted from the storage unit 1100 so as to be displayed at the head of an array of the image-related information pieces 610.

Operation S500 may include displaying extracted image-related information and an acquired ultrasonic image together.

For example, as illustrated in FIG. 8B, image-related information suitable for the ultrasonic image 230, for example, a body marker 620, may be displayed at the head of an array of body markers.

In other words, a body marker 620 located at a twenty second place from among a total of 25 body markers as illustrated in FIG. 8A may be a body marker that is the most suitable for the ultrasonic image 230, and the body marker 620 may be displayed at the head of an array of body markers as illustrated in FIG. 8B. For example, the body marker 620 may be displayed at the first place among a total of 25 body markers.

As illustrated in FIGS. 8A and 8B, since the body marker 620 is the most consistent with the ultrasonic image 230, a relation between the body marker 620 and the ultrasonic image 230 may be the highest.

According to an embodiment of the present invention, an image-related information piece having a highest relation with the ultrasonic image 230 may be displayed in preference to the other image-related information pieces.

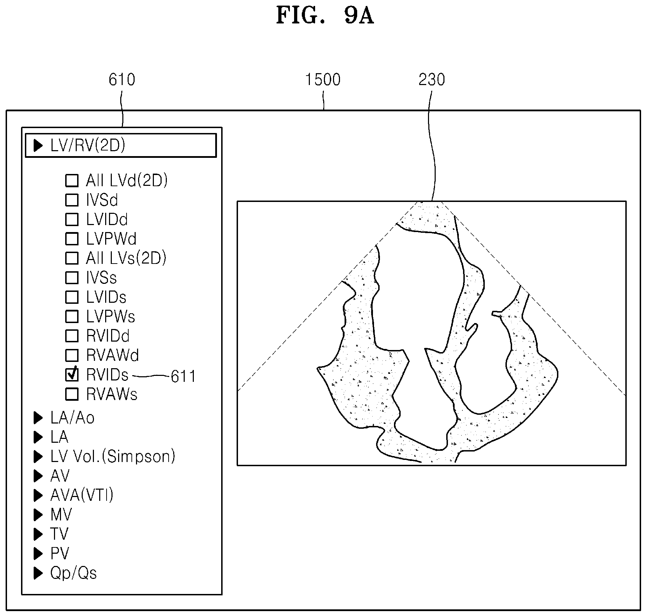

FIG. 9A illustrates another example of selection of information associated with an ultrasonic image 230.

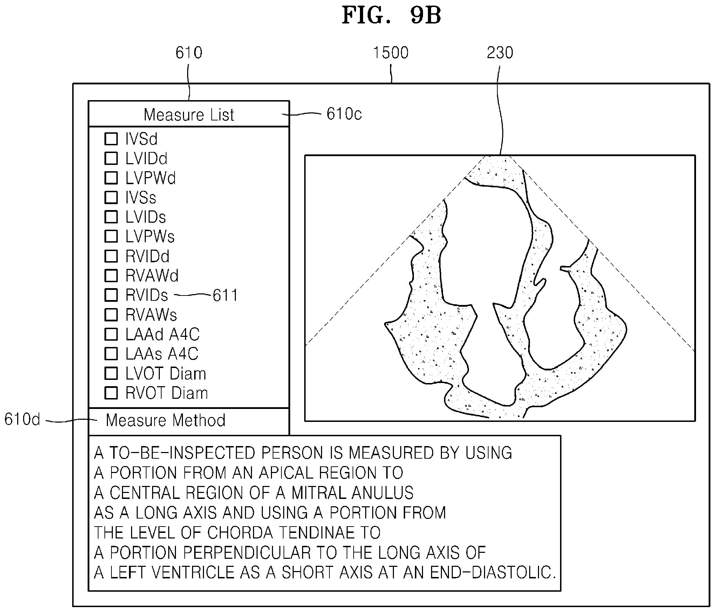

FIG. 9B illustrates a selective display of the information associated with the ultrasonic image 230, according to an embodiment of the present invention.

Similar to FIGS. 8A and 8B, in FIGS. 9A and 9B, the ultrasonic image 230 and the image-related information pieces 610 may be displayed. For example, the image-related information pieces 610 may be displayed together with the ultrasonic image 230 on the display unit 1500.

According to an embodiment of the present invention, as illustrated in FIG. 9B, the image-related information pieces 610 may be extracted starting from an image-related information piece having a highest relation with the ultrasonic image 230, and may be displayed in the order of the extraction.

The image-related information pieces 610 may include information about measuring items, standard measuring methods, and specific measuring methods. For example, the standard measuring methods may include a measuring method prescribed in a society such as the American Society of Echocardiography (ASE). The specific measuring methods may include a measuring method that is input to the apparatus 1000 according to an input signal of a user such as an organization or an individual person.

For example, if a target object is desired to be measured from the ultrasonic image 230, for example, if the length, the size, or the like of the target object is desired to be measured, information 610c and information 610d that are associated with the measurement of the target object may be extracted from the storage unit 1100 and displayed, according to the order of priority based on relations of the information 610c and the information 610d with the ultrasonic image 230.

For example, as illustrated in FIG. 9A, the apparatus 1000 may display basically-provided measurement items classified by category. Alternatively, as illustrated in FIG. 9B, measurement items 611 directly applicable to the ultrasonic image 230 may be extracted and displayed in the form of a list. The directly applicable measurement items 611 may be extracted based on the order of priority determined according to the relations of the directly applicable measurement items 611 with the ultrasonic image 230.

As illustrated in FIG. 9B, the image-related information pieces 610 may include the information 610d, which is associated with a measuring method.

The image-related information pieces 610 may be displayed using a text or using a picture or a moving picture. Alternatively, the image-related information pieces 610 may be displayed using at least one of a text and a moving picture.

The contents of the above-described apparatus 1000 may be equally applied to a method according to an embodiment of the present invention. Accordingly, descriptions of the above-described apparatus 1000 that are related to the method will not be repeated here.

The above-described embodiments of the present invention may be written as computer programs and may be implemented in general-use digital computers that execute the programs using a computer-readable recording medium.

Examples of the computer-readable recording medium include magnetic storage media (e.g., ROM, floppy disks, hard disks, etc.), optical recording media (e.g., CD-ROMs or DVDs), etc.

Up to now, the present invention has been described by referring to exemplary embodiments. While the exemplary embodiments have been particularly shown and described, it will be understood by those of ordinary skill in the art that various changes in form and details may be made therein s far as they fall under the scope of the present invention as defined by the appended claims. Therefore, the exemplary embodiments should be considered in descriptive sense only and not for purposes of limitation. Therefore, the scope of the present invention is defined not by the detailed description of exemplary embodiments, but by the appended claims.

## Claims

1. An apparatus (1000) for displaying an ultrasonic image (230) and information associated with the image, the apparatus (1000) comprising:
a storage unit (1100) which is configured to store a plurality of cross-section images (220) of a plurality of scanned cross-sections of a target object (210) and a plurality of pieces of image-related information respectively associated with the plurality of cross-section images (220);
an image acquisition unit (1200) which acquires an ultrasonic image (230) of the target object (210);
a selection unit (1300) which is configured to select at least one of the plurality of cross-section images (220) based on a similarity between the ultrasonic image (230) and each of the plurality of cross-section images (220);
an extraction unit (1400) which is configured to extract image-related information associated with the selected cross-section image from the storage unit (1100); and
a display unit (1500) which is configured to display the extracted image-related information and the acquired ultrasonic image (230),
wherein the image-related information pieces comprise at least one of information about a scan method for the target object (210), information about a biopsy of the target object (210), information about a measurement item of the target object (210), information about a measuring standard scales of the target object measurement (210) and information about a display of the target object,
wherein the selection unit (1300) comprises a comparison unit which is configured to determine a similarity between the acquired ultrasonic image (230) and each of the plurality of cross-section images (220) by comparing the acquired ultrasonic image (230) with each of the plurality of cross-section images (220), and the selected cross-section image is a cross-section image having a highest similarity to the acquired ultrasonic image (230) from among the plurality of cross-section images (220) **characterised in that**
the extraction unit (1400) comprises a determination unit which is configured to determine priorities of the image-related information pieces based on relations of the image-related information pieces with the selected cross-section image, and the extraction unit (1400) is configured to extract the image-related information pieces according to the determined priorities, and
wherein the extraction unit (1400) is further configured to extract as image-related information pieces, measurement items directly applicable to the acquired ultrasonic image (230), based on priorities determined according to relations of the measurement items with the selected cross-section image (230),
wherein the display unit (1500) is configured to display a list in which the measurement items directly applicable to the acquired ultrasonic image (230) are listed based on the priorities.

2. The apparatus (1000) of claim 1, wherein the plurality of cross-section images (220) comprise respective standard cross-section images (220) of a plurality of standard cross-sections.

3. The apparatus (1000) of claim 1, wherein
the comparison unit comprises an alarm unit which produces an alarm message when the determined similarity is less than or equal to a predetermined standard level, and
the alarm message is a message that advises the apparatus (1000) to re-acquire the ultrasonic image (230) of the target object (210), or a message that advises the apparatus (1000) to adjust the resolution of the ultrasonic image (230).

4. The apparatus (1000) of claim 1, wherein the display unit (1500) displays the extracted image-related information and the acquired ultrasonic image (230) together.

5. A method of displaying an ultrasonic image (230) and information associated with the image, the method comprising:
storing a plurality of cross-section images (220) of a plurality of scanned cross-sections of a target object (210) and a plurality of pieces of image-related information respectively associated with the plurality of cross-section images (220);
acquiring an ultrasonic image (230) of the target object (210);
selecting at least one of the plurality of cross-section images (220) based on a similarity between the ultrasonic image (230) and each of the plurality of cross-section images (220);
extracting image-related information associated with the selected cross-section image; and
displaying the extracted image-related information and the acquired ultrasonic image (230) together,
wherein the image-related information pieces comprise at least one of information about a scan method for the target object (210), information about a biopsy of the target object (210), information about a measurement item of the target object (210), information about a measuring standard scales of the target object measurement (210) and information about a display of the target object,
wherein the selecting comprises determining a similarity between the acquired ultrasonic image (230) and each of the plurality of cross-section images (220) by comparing the acquired ultrasonic image (230) with each of the plurality of cross-section images (220), and the selected cross-section image is a cross-section image having a highest similarity to the acquired ultrasonic image (230) from among the plurality of cross-section images (220) **characterised in that**
the extracting comprises determining priorities of the image-related information pieces based on relations of the image-related information pieces with the selected cross-section image, and the image-related information pieces are extracted according to the determined priorities,
wherein the extracting comprises extracting as image-related information pieces, measurement items directly applicable to the acquired ultrasonic image (230), based on priorities determined according to relations of the measurement items with the selected cross-section image (230),
wherein the displaying comprises displaying a list in which the measurement items directly applicable to the acquired ultrasonic image (230) are listed based on the priorities.

6. The method of claim 5, wherein the plurality of cross-section images (220) comprise respective standard cross-section images (220) of a plurality of standard cross-sections.

7. The method of claim 5, wherein
the determining comprises producing an alarm message when the determined similarity is less than or equal to a predetermined standard level, and
the alarm message is a message that advises the ultrasonic image (230) of the target object (210) to be re-acquired, or a message that advises the resolution of the ultrasonic image (230) to be adjusted.

8. The method of claim 5, wherein the displaying comprises displaying the extracted image-related information and the acquired ultrasonic image (230) together.

9. A non-transitory computer-readable recording medium having recorded thereon a program for executing the method of any of claims 5 through 8.

## Patentansprüche

1. Vorrichtung (1000) zum Anzeigen eines Ultraschallbilds (230) und mit dem Bild verbundene Informationen, wobei die Vorrichtung (1000) Folgendes aufweist:
eine Speichereinheit (1100), die dafür vorgesehen ist, eine Vielzahl von Querschnittsbildern (220) einer Vielzahl von eingescannten Querschnitten eines Zielobjekts (210) und eine Vielzahl von Teilen von sich auf das Bild beziehenden bzw. den bildbezogenen Informationen, die jeweils mit der Vielzahl von Querschnittsbildern (220) verbunden sind, zu speichern;
eine Bilderlangungseinheit (1200), die ein Ultraschallbild (230) von dem Zielobjekt (210) erlangt;
eine Auswahleinheit (1300), die dafür vorgesehen ist, wenigstens eines der Vielzahl von Querschnittsbildern (220) basierend auf einer Ähnlichkeit zwischen dem Ultraschallbild (230) und jedem der Vielzahl von Querschnittsbildern (220) auszuwählen;
eine Extraktionseinheit (1400), die dafür vorgesehen ist, bildbezogene Informationen, die mit dem ausgewählten Querschnittsbild in Verbindung stehen, aus der Speichereinheit (1100) zu extrahieren; und
eine Anzeigeeinheit (1500), die dafür vorgesehen ist, die extrahierten, bildbezogenen Informationen und das erlangte Ultraschallbild (230) anzuzeigen,
wobei die bildbezogenen Informationsteile wenigstens eines einer Information über ein Scanverfahren für das Zielobjekt (210), Informationen bezüglich einer Biopsie des Zielobjekts (210), Informationen bezüglich eines Messgegenstands des Zielobjekts (210), Informationen bezüglich eines Messstandardmaßstabs der Zielobjektmessung (210) und Informationen bezüglich einer Anzeige des Zielobjekts aufweisen,
wobei die Auswahleinheit (1300) eine Vergleichseinheit aufweist, die dafür vorgesehen ist, eine Ähnlichkeit zwischen dem erlangten Ultraschallbild (230) und jedem der Vielzahl von Querschnittsbildern (220) durch Vergleichen des erlangten Ultraschallbilds (230) mit jedem der Vielzahl von Querschnittsbildern (220) zu ermitteln, und wobei das ausgewählte Querschnittsbild ein Querschnittsbild ist, das eine höchste Ähnlichkeit mit dem erlangten Ultraschallbild (230) aus der Vielzahl von Ultraschallbildern aufweist,
**dadurch gekennzeichnet, dass**
die Extraktionseinheit (1400) eine Ermittlungseinheit aufweist, die dafür vorgesehen ist, Prioritäten der bildbezogenen Informationsteile basierend auf Beziehungen der bildbezogenen Informationsteile mit dem ausgewählten Querschnittsbild zu ermitteln, und dass die Extraktionseinheit (1400) dafür vorgesehen ist, die bildbezogenen Informationsteile unter den ermittelten Prioritäten zu extrahieren, und
wobei die Extraktionseinheit (1400) des Weiteren dafür vorgesehen ist, als bildbezogene Informationsteile Messgegenstände, die direkt auf das erlangte Ultraschallbild (230) anwendbar sind, basierend auf Prioritäten, die gemäß Beziehungen der Messgegenstände zu dem ausgewählten Querschnittsbild (230) ermittelt wurden, zu extrahieren,
wobei die Anzeigeeinheit (1500) dafür vorgesehen ist, eine Liste anzuzeigen, in welcher die Messgegenstände, die direkt auf das erlangte Ultraschallbild (230) anwendbar sind, basierend auf den Prioritäten gelistet sind.

2. Vorrichtung (1000) nach Anspruch 1, wobei die Vielzahl von Querschnittsbildern (220) jeweilige Standardquerschnittsbilder (220) einer Vielzahl von Standardquerschnitten aufweist.

3. Vorrichtung (1000) nach Anspruch 1, wobei die Vergleichseinheit eine Alarmeinheit aufweist, die eine Alarmnachricht erzeugt, wenn die ermittelte Ähnlichkeit weniger oder gleich einem vorbestimmten Standardniveau ist, und die Alarmnachricht eine Nachricht ist, welche die Vorrichtung (100) anweist, das Ultraschallbild (230) des Zielobjekts (210) wiederzuerlangen, oder eine Nachricht, die die Vorrichtung (1000) anweist, die Auflösung des Ultraschallbilds (230) anzupassen.

4. Vorrichtung (1000) nach Anspruch 1, wobei die Anzeigeeinheit (1500) die extrahierten, bildbezogenen Informationen und das erlangte Ultraschallbild (230) gemeinsam anzeigt.

5. Verfahren zum Anzeigen eines Ultraschallbilds (230) und mit dem Bild verbundenen Informationen, wobei das Verfahren Folgendes aufweist:
Speichern einer Vielzahl von Querschnittsbildern (220) einer Vielzahl von gescannten Querschnitten eines Zielobjekts (210) und eine Vielzahl von Teilen von sich auf das Bild beziehenden bzw. bildbezogenen Informationen, die jeweils mit der Vielzahl von Querschnittsbildern (220) verbunden sind;
Erlangen eines Ultraschallbilds (230) des Zielobjekts (210);
Auswählen wenigstens eines der Vielzahl von Querschnittsbildern (220) basierend auf einer Ähnlichkeit zwischen dem Ultraschallbild (230) und jedem der Vielzahl von Querschnittsbildern (220);
Extrahieren von bildbezogenen Informationen, die mit dem ausgewählten Querschnittsbild in Verbindung stehen; und
gemeinsames Anzeigen der extrahierten, bildbezogenen Informationen und des erlangten Ultraschallbild (230),
wobei die bildbezogenen Informationsteile wenigstens eines einer Information über ein Scanverfahren für das Zielobjekt (210), Informationen bezüglich einer Biopsie des Zielobjekts (210), Informationen bezüglich eines Messgegenstands des Zielobjekts (210), Informationen bezüglich eines Messstandardmaßstabs der Zielobjektmessung (210) und Informationen bezüglich einer Anzeige des Zielobjekts aufweisen,
wobei das Auswählen das Ermitteln einer Ähnlichkeit zwischen dem erlangten Ultraschallbild (230) und jedem der Vielzahl von Querschnittsbildern (220) durch Vergleichen des erlangten Ultraschallbilds (230) mit jedem der Vielzahl von Querschnittsbildern (220) aufweist, und wobei das ausgewählte Querschnittsbild ein Querschnittsbild ist, das das eine höchste Ähnlichkeit mit dem erlangten Ultraschallbild (230) aus der Vielzahl von Ultraschallbildern (220) aufweist,
**dadurch gekennzeichnet, dass**
das Extrahieren das Ermitteln von Prioritäten der bildbezogenen Informationsteile basierend auf Beziehungen der bildbezogenen Informationsteile mit dem ausgewählten Querschnittsbild aufweist, und dass die bildbezogenen Informationsteile gemäß den ermittelten Prioritäten extrahiert werden,
wobei das Extrahieren das Extrahieren von Messgegenständen, die direkt an den erlangten Ultraschallbildern (230) anwendbar sind, als bildbezogene Informationsteile aufweist, und zwar basierend auf Prioritäten, die gemäß den Beziehungen der Messgegenstände mit dem ausgewählten Querschnittsbild (230) ermittelt werden,
wobei das Anzeigen das Anzeigen einer Liste aufweist, in welcher die Messgegenstände, die direkt auf das erlangte Ultraschallbild (230) anwendbar sind, basierend auf den Prioritäten aufgelistet sind.

6. Verfahren nach Anspruch 5, wobei die Vielzahl von Querschnittsbildern (220) jeweilige Standardquerschnittsbilder (220) einer Vielzahl von Standardquerschnitten aufweist.

7. Verfahren nach Anspruch 5, wobei das Ermitteln das Erzeugen einer Alarmnachricht aufweist, wenn die ermittelte Ähnlichkeit weniger oder gleich einem vorbestimmten Standardniveau ist, und
die Alarmnachricht eine Nachricht ist, die anweist, dass das Ultraschallbild (230) des Zielobjekts (210) wiedererlangt wird, oder eine Nachricht, die anweist, die Auflösung des Ultraschallbilds (230) einzustellen.

8. Verfahren nach Anspruch 5, wobei das Anzeigen das gemeinsame Anzeigen der extrahierten bildbezogenen Informationen und des erlangten Ultraschallbilds (230) aufweist.

9. Nicht flüchtiges, computerlesbares Aufzeichnungsmedium, auf dem ein Programm zur Durchführung des Verfahrens nach einem der Ansprüche 5 bis 8 aufgezeichnet ist.

## Revendications

1. Appareil (1000) pour afficher une image ultrasonore (230) et
des informations associées à l'image, l'appareil (1000) comprenant :
une unité de stockage (1100) qui est configurée pour stocker une pluralité d'images en coupe transversale (220) d'une pluralité de coupes transversales numérisées d'un objet cible (210) et une pluralité d'éléments d'informations liés à l'image associés respectivement à la pluralité d'images en coupe transversale (220) ;
une unité d'acquisition d'image (1200) qui acquiert une image ultrasonore (230) de l'objet cible (210) ;
une unité de sélection (1300) qui est configurée pour sélectionner au moins une parmi la pluralité d'images en coupe transversale (220) sur la base d'une similitude entre l'image ultrasonore (230) et chacune de la pluralité d'images en coupe transversale (220) ;
une unité d'extraction (1400) qui est configurée pour extraire des informations liées à l'image associées à l'image en coupe transversale sélectionnée à partir de l'unité de stockage (1100) ; et
une unité d'affichage (1500) qui est configurée pour afficher les informations liées à l'image extraites et l'image ultrasonore acquise (230),
dans lequel les éléments d'informations liés à l'image comprennent au moins une information parmi un procédé de numérisation pour l'objet cible (210), des informations sur une biopsie de l'objet cible (210), des informations sur un élément de mesure de l'objet cible (210), des informations sur une échelle de mesure standard de l'objet cible (210) et des informations sur un affichage de l'objet cible,
dans lequel l'unité de sélection (1300) comprend une unité de comparaison qui est configurée pour déterminer une similitude entre l'image ultrasonore acquise (230) et chacune de la pluralité d'images en coupe transversale (220) en comparant l'image ultrasonore acquise (230) avec chacune de la pluralité d'images en coupe transversale (220), et l'image en coupe transversale sélectionnée est une image en coupe transversale ayant une similitude la plus élevée avec l'image ultrasonore acquise (230) parmi la pluralité d'images en coupe transversale (220), **caractérisé en ce que**
l'unité d'extraction (1400) comprend une unité de détermination qui est configurée pour déterminer des priorités des éléments d'informations liés à l'image sur la base de relations des éléments d'informations liés à l'image avec l'image en coupe transversale sélectionnée, et l'unité d'extraction (1400) est configurée pour extraire les éléments d'informations liés à l'image selon les priorités déterminées, et
dans lequel l'unité d'extraction (1400) est en outre configurée pour extraire, en tant qu'éléments d'informations liés à l'image, des éléments de mesure directement applicables à l'image ultrasonore acquise (230), sur la base de priorités déterminées en fonction de relations des éléments de mesure avec l'image en coupe transversale sélectionnée (230),
dans lequel l'unité d'affichage (1500) est configurée pour afficher une liste dans laquelle les éléments de mesure directement applicables à l'image ultrasonore acquise (230) sont répertoriés sur la base des priorités.

2. Appareil (1000) selon la revendication 1, dans lequel la pluralité d'images en coupe transversale (220) comprend des images en coupe transversale standard respectives (220) d'une pluralité de coupes transversales standard.

3. Appareil (1000) selon la revendication 1, dans lequel
l'unité de comparaison comprend une unité d'alarme qui produit un message d'alarme lorsque la similitude déterminée est inférieure ou égale à un niveau standard prédéterminé, et
le message d'alarme est un message qui conseille à l'appareil (1000) de réacquérir l'image ultrasonore (230) de l'objet cible (210), ou un message qui conseille à l'appareil (1000) d'ajuster la résolution de l'image ultrasonore (230).

4. Appareil (1000) selon la revendication 1, dans lequel l'unité d'affichage (1500) affiche ensemble les informations liées à l'image extraites et l'image ultrasonore acquise (230).

5. Méthode d'affichage d'une image ultrasonore (230) et d'informations associées à l'image, la méthode comprend:
le stockage d'une pluralité d'images en coupe transversale (220) d'une pluralité de coupes transversales numérisées d'un objet cible (210) et une pluralité d'éléments d'informations liés à l'image respectivement associés à la pluralité d'images en coupe transversale (220) ;
l'acquisition d'une image ultrasonore (230) de l'objet cible (210) ;
la sélection d'au moins une parmi la pluralité d'images en coupe transversale (220) sur la base d'une similitude entre l'image ultrasonore (230) et chacune de la pluralité d'images en coupe transversale (220) ;
l'extraction des informations liées à l'image associées à l'image en coupe transversale sélectionnée ; et
l'affichage de l'ensemble les informations liées à l'image extraites et l'image ultrasonore acquise (230),
dans laquelle les éléments d'informations liés à l'image comprennent au moins une information parmi un procédé de numérisation pour l'objet cible (210), des informations sur une biopsie de l'objet cible (210), des informations sur un élément de mesure de l'objet cible (210), des informations sur une échelle de mesure standard de l'objet cible (210) et des informations sur un affichage de l'objet cible,
dans laquelle la sélection comprend la détermination d'une similitude entre l'image ultrasonore acquise (230) et chacune de la pluralité d'images en coupe transversale (220) en comparant l'image ultrasonore acquise (230) avec chacune de la pluralité d'images en coupe transversale (220), et l'image en coupe transversale sélectionnée est une image en coupe transversale ayant une similitude la plus élevée avec l'image ultrasonore acquise (230) parmi la pluralité d'images en coupe transversale (220), **caractérisée en ce que**
l'extraction comprend la détermination de priorités des éléments d'informations liés à l'image sur la base de relations des éléments d'informations liés à l'image avec l'image en coupe transversale sélectionnée, et les éléments d'informations liés à l'image sont extraits selon les priorités déterminées, et
dans laquelle l'extraction comprend l'extraction, en tant qu'éléments d'informations liés à l'image, des éléments de mesure directement applicables à l'image ultrasonore acquise (230), sur la base de priorités déterminées en fonction de relations des éléments de mesure avec l'image en coupe transversale sélectionnée (230),
dans laquelle l'affichage comprend l'affichage d'une liste dans laquelle les éléments de mesure directement applicables à l'image ultrasonore acquise (230) sont répertoriés sur la base des priorités.

6. Méthode selon la revendication 5, dans laquelle la pluralité d'images en coupe transversale (220) comprend des images en coupe transversale standard respectives (220) d'une pluralité de coupes transversales standard.

7. Méthode selon la revendication 5, dans laquelle
la détermination comprend la production d'un message d'alarme lorsque la similitude déterminée est inférieure ou égale à un niveau standard prédéterminé, et
le message d'alarme est un message qui conseille de réacquérir l'image ultrasonore (230) de l'objet cible (210), ou un message qui conseille d'ajuster la résolution de l'image ultrasonore (230).

8. Méthode selon la revendication 5, dans laquelle l'affichage comprend l'affichage ensemble des informations liées à l'image extraites et de l'image ultrasonore acquise (230).

9. Support d'enregistrement non transitoire lisible par ordinateur sur lequel est enregistré un programme pour exécuter la méthode selon l'une quelconque des revendications 5 à 8.
